(19) Europäisches Patentamt
European Patent Office
Office européen des brevets

(11) Veröffentlichungsnummer: **0 300 234 B1**

# (12) EUROPÄISCHE PATENTSCHRIFT

(45) Veröffentlichungstag der Patentschrift: **06.05.92**

(51) Int. Cl.⁵: **C08B 37/08**, A61K 7/043

(21) Anmeldenummer: **88110263.6**

(22) Anmeldetag: **28.06.88**

## (54) Nagellack auf der Basis von O-Benzyl-N-hydroxyalkylchitosanen sowie neue O-Benzyl-N-hydroxyalkylderivate des Chitosans.

(30) Priorität: **18.07.87 DE 3723811**

(43) Veröffentlichungstag der Anmeldung:
**25.01.89 Patentblatt 89/04**

(45) Bekanntmachung des Hinweises auf die Patenterteilung:
**06.05.92 Patentblatt 92/19**

(84) Benannte Vertragsstaaten:
**DE ES FR GB IT**

(56) Entgegenhaltungen:
**EP-A- 0 193 736**

**CHEMICAL ABSTRACTS, Band 98, Nr. 22, Mai 1983, Seite 105, Zusammenfassung Nr. 181486e, Columbus, Ohio, US; & JP-A-57 180 602 (NIPPON SODA CO., LTD) 06-11-1982**

**MANUFACTURING CHEMIST, Band 55, Nr. 10, Oktober 1984, Seiten 47,49,52, Poole, Dorset, GB; E.S. LOWER: "Polymers from the sea chitin & chitosan"**

(73) Patentinhaber: **Wella Aktiengesellschaft**
**Berliner Allee 65**
**W-6100 Darmstadt(DE)**

(72) Erfinder: **Lang, Günther Dr.**
**Auf der Roten Erde 10 B**
**W-6107 Reinheim 5(DE)**
Erfinder: **Maresch, Gerhard**
**Winkelschneise 6**
**W-6100 Darmstadt(DE)**
Erfinder: **Lenz, Hans-Rudi**
**Moltkestrasse 16**
**W-6100 Darmstadt(DE)**

EP 0 300 234 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Wesentliche Eigenschaften, die einen guten Nagellack auszeichnen sollten, sind ausreichende Härte, gute Streichfähigkeit, kurze Trockenzeit, hohe Lagerstabilität (das bedeutet, daß der Nagellack seine Homogenität und eine gute Stabilität über einen längeren Zeitraum beibehält), Beständigkeit gegen Licht, Wasser, Wasch- und Spülmittel sowie vor allem Unschädlichkeit gegenüber Haut und Nägeln. Schließlich sollte der Nagellack einen Lackfilm mit zufriedenstellenden Eigenschaften ergeben. Die von einem solchen Nagellackfilm erwarteten Eigenschaften sind eine gleichmäßige Dicke, ein hoher Glanz, was eine glatte Oberfläche voraussetzt, ein hervorragendes Haftvermögen auf dem Keratin das Nagels und eine gute Elastizität. Letztere verhindert, daß der Lack bricht oder abblättert.

Nagellacke enthalten im allgemeinen einen Filmbildner, eine Harzkomponente und ein Lösungsmittelsystem sowie gegebenenfalls Weichmacher, Pigmente und übliche Zusätze.

Übliche Nagellacke enthalten als Filmbildner Nitrocellulose. Besonders bevorzugt werden hierbei esterlösliche Nitrocellulosen (sogenannte E-Collodiumwolle oder "RS"-Nitrocellulose) mit einer mittleren bis niedrigen Viskosität. Nagellacke auf Nitrocellulosebasis besitzen jedoch eine Vielzahl von Nachteilen: So können zum Beispiel bei Verwendung von Nitrocellulose mit der Zeit Verfärbungen des Nagellacks auftreten. Weiterhin neigt Nitrocellulose zu plötzlichen Viskositätsänderungen, wodurch ein gleichmäßiges Auftragen des Nagellackes erschwert wird. Außerdem muß darauf geachtet werden, daß die in Nagellacken verwendete Nitrocellulose neutral, das heißt frei von Säurebestandteilen, ist. Ein Gehalt an freier Säure kann nämlich sowohl zu einer Schädigung der Fingernägel und der Haut als auch zur Zerstörung der in den Nagellacken enthaltenen Farbstoffe führen.

Ferner ist bekannt, daß Nitrocellulose wegen ihrer großen Feuer- und Explosionsgefährlichkeit mit größter Vorsicht hergestellt und gehandhabt werden muß.

Um zufriedenstellende Ergebnisse bezüglich Haftfestigkeit, Glanz und Härte des Nagellacks zu erhalten, müssen modernen Kombinationslacken auf Nitrocellulosebasis noch verschiedene andere Harzkomponenten zugesetzt werden. Hierbei kommen neben natürlichen Polymeren, wie zum Beispiel Schellack, Elemi und insbesondere Kolophonium, Kunstharze, wie beispielsweise Polystyrol, Polyvinylacetat und Polymethacrylsäureester, zum Beispiel Polypropylmethacrylat und Polymethylmethacrylat, zum Einsatz. Ferner werden Alkydharze, wie zum Beispiel Polymerisate aus Phtalsäureanhydrid und Glycerin, sowie Formaldehyd/Harnstoff-Harze und bevorzugt Arylsulfonamid/Formaldehyd-Harze, wie zum Beispiel ein aus äquimolaren Mengen von Formaldehyd und p-Toluolsulfonamid hergestelltes Polymer, welches unter der Bezeichnung Santolite® bekannt ist, eingesetzt.

Es wurde bereits mehrfach versucht, die oben genannten Nachteile durch Verwendung von synthetischen Copolymeren, wie zum Beispiel Copolymerisaten aus hydrophoben und hydrophilen Monomereinheiten (siehe EP-OS 00 85 370), anstelle von Nitrocellulose als Filmbildner in Nagellacken zu vermeiden. Weiterhin sei auf die deutschen Offenlegungsschriften DE-OS 31 12 888 und DE-OS 32 05 545 verwiesen.

Trotz aller bisherigen Bemühungen, nitrocellulosefreie Nagellacke herzustellen und obwohl ein ausschließlich auf Nitrocellulose basierender Nagellackfilm den erwähnten Anforderungen nicht genügt, und er erst durch Zusatz weiterer Harzkomponenten härter, haftfester, elastischer und widerstandsfähiger wird, stellt dieser Filmbildner einen nach wie vor unverzichtbaren Bestandteil vieler Nagellacke dar. Es ist nämlich bisher nicht gelungen, nitrocellulosefreie Nagellacke zu entwickeln, die in allen für Nagellacke wesentlichen Eigenschaften gleichgute oder sogar bessere Ergebnisse als Nagellacke auf Nitrocellulosebasis erzielen. So können sich beispielsweise bei den oben erwähnten, auf synthetischen Polymeren basierenden Nagellacken, wegen der physiologischen Wirkung von eventuell vorhandenen, nur schwer aus dem Polymerisat entfernbaren, Monomerspuren Probleme ergeben.

Im Gegensatz hierzu sind die in der eigenen DE-OS 35 37 333 für die Verwendung als Filmbildner in Nagellacken vorgeschlagenen Chitinalkylester physiologisch unbedenklich. Jedoch sind diese Chitinalkylester nur durch ein kompliziertes und teures Verfahren erhältlich. Insbesondere die Herstellung von hochmolekularen, hinreichend substituierten Chitinalkylestern, die auch ohne Zusatz von Weichmachern geschlossene Filmoberflächen bilden können, ist nur unter erheblichem technischen Aufwand und exakter Reaktionsführung bei 0 Grad Celsius möglich.

Aufgabe der vorliegenden Erfindung ist es, physiologisch gut verträgliche, nitrocellulosefreie Nagellacke zur Verfügung zu stellen, die gleichgute oder bessere Lackfilme als Nagellacke auf Nitrocellulosebasis ergeben und deren filmbildende Komponente durch eine technisch einfache und preiswerte Synthese erhältlich ist.

Überraschenderweise wurde nun gefunden, daß bei Verwendung von bestimmten, in organischen Lösungsmitteln löslichen, O-Benzyl-N-hydroxyalkylderivaten des Chitosans als Filmbildner anstelle von Nitrocellulose gleichgute oder bessere Lackfilme erhalten werden. Die mit auf diesen O-Benzyl-N-hydroxyalkylchitosanen basierenden Nagellacken erzielten Lackfilme zeichnen sich durch einen hohen Glanz, ein hervorragendes Haftvermögen und eine gegenüber Nagellacken auf Nitrocellulosebasis erhöhte Härte bei hinreichender Elastizität aus.

Weiterhin wurde gefunden, daß es möglich ist, die in Nagellacken auf Nitrocellulosebasis zusätzlich enthaltenen Harzkomponenten vorteilhaft durch die erfindungsgemäßen O-Benzyl-N-hydroxyalkylchitosane zu ersetzen. Hierdurch werden Nachteile, wie sie beispielsweise bei Verwendung von Arylsulfonamid/Formaldehyd-Harzen auftreten können (zum Beispiel eine mäßige Lichtbeständigkeit der Nagellacke, Freisetzen von Formaldehyd oder ein erhöhtes Risiko allergischer Reaktionen), vermieden.

Die Erfindung betrifft demzufolge einen Nagellack auf der Basis eines Filmbildners, einer Harzkomponente und eines Lösungsmittelsystems, welcher dadurch gekennzeichnet ist, daß er als Filmbildner und/oder Harzkomponente ein O-Benzyl-N-hydroxyalkylchitosan der allgemeinen Formel (I)

$$HO\,[\,C_6H_{11\text{-}m\text{-}q}NO_4\ (R^1)_m(R^2)_n(R^3)_q]_pH \qquad (I),$$

wobei m jeden beliebigen Zahlenwert von 0 bis 0,6,
n jeden beliebigen Zahlenwert von 0,1 vis 10
und q jeden beliebigen Zahlenwert von 0,1 bis 4
annehmen kann,
der Polymerisationsgrad p für eine ganze Zahl von 50 bis 5.000 steht,
$R^1$ den Rest

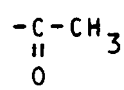

bedeutet,
$R^2$ den Rest

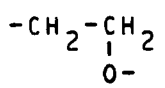 , 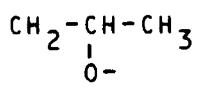 oder

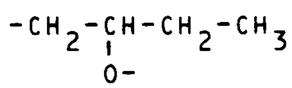

darstellt
und $R^3$ den Rest

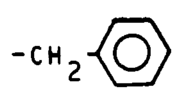

bedeutet; enthält.

Die in dem ergindungsgemäßen Nagellack enthaltenen O-Benzyl-N-hydroxyalkylchitosane haben vorzugsweise ein durchschnittliches Molekulargewicht von etwa 5.000 bis 1.800.000. Besonders bevorzugt ist hierbei ein durchschnittliches Molekulargewicht von etwa 10.000 bis 500.000, welches durch Molekularausschluß-Chromatographie an Ultrastyragelsäulen der Firma WATERS unter Verwendung von Tetrahydrofuran als Eluenten ermittelt wurde.

Die Grenzviskositätszahlen (Eta) der O-Benzyl-N-hydroxyalkylchitosane der Formel (I), ermittelt mit einem DIN-Ubbelohde-Viskosimeter bei 25 Grad Celsius in Tetrahydrofuran, liegen in einem Bereich von etwa 5 bis 200 ml/g, vorzugsweise 10 bis 120 ml/g.

Der aus $^1H$-NMR-Spektren ermittelte Benzyl-Substitutionsgrad der O-Benzyl-N-hydroxyalkylchitosane beträgt vorzugsweise 0,8 bis 3,8.

Die in dem erfindungsgemäßen Nagellack als Filmbildner und/oder Harzkomponente verwendeten O-Benzyl-N-hydroxyalkylchitosane der Formel (I) führen weder zu Hautreizungen, noch sind sie toxisch, sie sind physiologisch unbedenklich und biologisch abbaubar.

Erfindungsgemäß können die O-Benzyl-N-hydroxyalkylchitosane der Formel (I) je nach Einsatz als Filmbildner oder Harzkomponente in dem Nagellack alleine oder zusammen mit anderen Harzen, wie zum Beispiel Nitrocellulose, verwendet werden.

Die Konzentration der O-Benzyl-N-hydroxyalkylchitosane der Formel (I) beträgt bei Verwendung als Filmbildner etwa 3 bis 30 Gewichtsprozent. Ein Zusatz weiterer Harzkomponenten, wie dies bei Verwendung von Nitrocellulose als Filmbildner notwendig ist, ist hierbei nicht erforderlich.

Werden die O-Benzyl-N-hydroxyalkylchitosane der Formel (I) als Harzkompontente eingesetzt, so sind sie in einer Konzentration von etwa 10 bis 70 Gewichtsprozent, bezogen auf die Menge des enthaltenen Filmbildners, beziehungsweise in einer Konzentration von etwa 1 bis 21 Gewichtsprozent, bezogen auf das Gesamtgewicht des Nagellackes, in dem erfindungsgemäßen Nagellack enthalten.

Der erfindungsgemäße Nagellack ergibt bei geeigneter Formulierung auch ohne Zusatz von Weichmachern einen hochglänzenden, glatten, transparenten, zusammenhängenden Film. Jedoch kann es in bestimmten Fällen vorteilhaft sein, dem Nagellack einen oder mehrere Weichmacher in einer Gesamtmenge von etwa 0,2 bis 12 Gewichtsprozent zuzusetzen, um dem Lackfilm eine erhöhte Geschmeidigkeit und Elastizität zu verleihen, seine Neigung zum Schrumpfen herabzusetzen sowie die Haftfestigkeit auf der Nagelplatte und den Glanz zu verbessern. Die verwendeten Weichmacher sollen hochsiedend, das heißt nicht flüchtig, mit dem Filmbildner und den übrigen Bestandteilen des Nagellackes mischbar, farblos, geruchlos sowie ungiftig sein. Verwendung finden Ester mehrbasiger Säuren, wie zum Beispiel Dibutylphthalat, Diisobutylphthalat, Diamylphthalat, Dioctylphthalat, Dimethoxyethylphthalat, Tributylphosphat, Triphenylphosphat, Trikresylphosphat, Tributoxyethylphosphat, Triethylcitrat, Tributylcitrat, Tributylacetylcitrat und Dibutyltartrat, Ester gesättigter und ungesättigter Fettsäuren, wie beispielsweise Butylstearat, Butylacetylricinoleat und Glycerylacetylricinoleat, Ricinusöl, Campher sowie Mischungen aus diesen Verbindungen.

Weiterhin enthält der erfindungsgemäße Nagellack ein Lösungsmittelsystem in einer Konzentration von etwa 60 bis 80 Gewichtsprozent.

Die Bezeichnung "Lösungsmittelsystem" steht für eine Mischung aus niedrig-, mittel- und hochsiedenden organischen Lösungsmitteln, die eine gute Verstreichbarkeit und relativ kurze Trocknungszeit des Lackfilms ermöglicht.

Als niedrigsiedende Lösungsmittel können Lösungsmittel mit einem Siedepunkt von etwa 30 bis 100 Grad Celsius, wie zum Beispiel Ethanol, Isopropanol, Aceton, Methylenchlorid, Ethylacetat und Methylacetat, verwendet werden. Als mittelsiedende Lösungsmittel werden Lösungsmittel mit einem Siedepunkt von etwa 100 bis 150 Grad Celsius, wie zum Beispiel Butanol, Amylalkohol, Toluol, Ethylenglykolmonomethylether, Butylacetat und Amylacetat, eingesetzt. Als hochsiedende Lösungsmittel kommen Lösungsmittel mit einem Siedepunkt von etwa 150 bis 200 Grad Celsius, beispielsweise Dichlorethylether, Diethylenglykolmonoethylether, Diethylenglykolmonomethylether und Ethylenglykol, in Betracht.

Der erfindungsgemäße Nagellack kann auch gefärbt vorliegen. Er enthält dann mindestens ein organisches oder anorganisches Pigment, vorzugsweise in einer Menge von etwa 0,1 bis 6 Gewichtsprozent. Als organische Pigmente können insbesondere Calcium-, Aluminium- und Lithol-Farblacke, wie zum Beispiel der Aluminiumlack von FD & C Gelb No. 5 (C.I. 19 140:1), der Aluminiumlack von FD & C Gelb No. 6 (C.I. 15 885:1), Lithol Rubin B (C.I. 15 850) und die Lithollacke D & C Rot No. 10, 11, 12 und 13 (C.I. 15 630), sowie Guanin (C.I. 75 170) genannt werden. Weitere in den erfindungsgemäßen Nagellacken verwendbare Farblacke, wie zum Beispiel der Aluminiumlack von D & C Rot No. 7, der Calciumlack von D & C Rot No. 7 und der Calciumlack von D & C Rot No. 34, werden im CTFA Cosmetic Ingredient Dictionary (1982), The Cosmetic, Toiletry und Fragrance Associoation, Inc., Washington DC/USA, beschrieben.

Als anorganische Pigmente können beispielsweise braunes und rotes Eisenoxid, Titandioxid und Wismutoxidchlorid eingesetzt werden.

Der erfindungsgemäße Nagellack kann wieterhin alle für einen Nagellack üblichen Bestandteile enthalten, wie beispielsweise Parfümöle oder Zusätze, die eine Sedimentation verzögern. Als Sedimentationsverzögerer werden insbesondere siliciumdioxidhaltige Verbindungen, wie zum Beispiel kolloidale Kieselsäure oder Tone des Montmorillonit-Typs (zum Beispiel Bentone 27 und Bentone 38 der National Lead Co.), sowie Metallseifen, wie beispielsweise Aluminium- und Zinkstearat, eingesetzt.

Die in dem erfindungsgemäßen Nagellack enthaltenen neuen O-Benzyl-N-hydroxyalkylchitosane der Formel (I) sind physiologisch unbedenklich und biologisch abbaubar. Neben der Verwendung in Nagellakken sind die neuen O-Benzyl-N-hydroxyalkylchitosane auch für die Verwendung in Klebstoffen und Lacken, in Pharmazeutika und Kosmetika sowie bei der Folien- und Filmherstellung geeignet.

Gegenstand der vorliegenden Erfindung sind daher weiterhin neue makromolekulare, vom Chitosan abgeleitete O-Benzyl-N-hydroxyalkylverbindungen der allgemeinen Formel (I)

$$HO\,[\,C_6H_{11-m-q}NO_4\,(R^1)_m\,(R^2)_n\,(R^3)_q\,]_p\,H \qquad (I),$$

wobei m jeden beliebigen Zahlenwert von 0 bis 0,6,
n jeden beliebigen Zahlenwert von 0,1 bis 10
und q jeden beliebigen Zahlenwert von 0,1 bis 4
annehmen kann,
p für eine ganze Zahl von 50 bis 5.000 steht,
$R^1$ den Rest

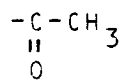

bedeutet,
$R^2$ den Rest

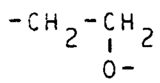 , 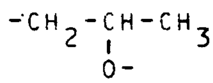 oder

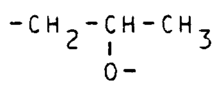

darstellt
und $R^3$ den Rest

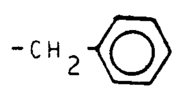

bedeutet.

Die neuen O-Benzyl-N-hydroxyalkylchitosane der Formel (I) werden erhalten, indem man Chitosan entweder in einer Zweistufenreaktion zunächst mit einem Epoxid und dann mit Benzylchlorid umsetzt oder beide Alkylierungsmittel gleichzeitig, jedoch bei zwei verschiedenen Reaktionstemperaturen, auf das Chitosan einwirken läßt.

Bei dem zweistufigen Verfahren zur Herstellung der Verbindungen der allgemeinen Formel (I) setzt man Chitosan, bestehend aus zu 40 bis 96 Prozent deacetyliertem Chitin, in einer 1. Reaktionsstufe in Gegenwart eines geeigneten Dispergiermittels bei einer Temperatur von 20 bis 120 Grad Celsius, vorzugsweise 80 bis 100 Grad Celsius, 6 bis 60 Stunden lang mit einem $C_2$ bis $C_4$ - Epoxid (Ethylenoxid, Propylenoxid, Butylenoxid) um und bringt anschließend das erhaltene N-Hydroxyalkylchitosan in einer 2. Reaktionsstufe mit Benzylchlorid im alkalischen Medium bei 40 bis 120 Grad Celsius, vorzugsweise bei 60 bis 90 Grad Celsius, 6 bis 60 Stunden lang zur Umsetzung.

Bei der gleichzeitigen Umsetzung des Chitosans mit beiden Alkylierungsmitteln (Epoxid und Benzylchlorid) wird das Reaktionsgemisch zunächst 6 bis 60 Stunden lang bei 20 bis 120 Grad Celsius, vorzugsweise bei 20 bis 40 Grad Celsius, im Autoklaven gerührt. Unter diesen Reaktionsbedingungen erfolgt überwiegend eine N-Hydroxyalkylierung des Chitosans. Anschließend wird das Reaktionsgemisch auf einen alkalischen pH-Wert eingestellt und bei 40 bis 120 Grad Celsius, vorzugsweise bei 60 bis 90 Grad Celsius, 6 bis 60 Stunden lang weitergerührt. Hierbei erfolgt die O-Alkylierung des N-Hydroxyalkylchitosans.

Das molare Verhältnis von Chitosan und Alkylierungsmittel wählt man jeweils zwischen 1:3 und 1:5.

Als Dispergiermittel werden bei niedrigen Reaktionstemperaturen die als Alkylierungsmittel eingesetzten Verbindungen verwendet. Bei höheren Temperaturen werden als Dispergiermittel organische Lösungsmittel, wie zum Beispiel Isopropanol, tert. Butanol, Ethylenglykoldimethylether, Dioxan und Toluol, verwendet.

Die Aufarbeitung der erhaltenen Reaktionsgemische erfolgt in der Weise, daß man nach Beseitigung des überschüssigen Epoxids das hydroxyalkylierte Zwischenprodukt durch Einengen aus dem organischen Lösungsmittel isoliert beziehungsweise nach der O-Benzylierung im alkylischen Medium das Reaktionsgemisch zunächst neutralisiert, sodann einengt und die anorganischen Salze durch Dekantieren, Zentrifugieren

oder Filtrieren abtrennt. Anschließend wird das Chitosanderivat zur Entfernung von Glykolen oder Glykolethern in Aceton oder Petrolether ausgefällt und fein dispergiert. Sodann wird das Chitosanderivat abfiltriert und getrocknet.

Die nachfolgenden Beispiele sollen den Gegenstand der Erfindung näher erläutern, ohne diesen hierauf zu beschränken.

**Herstellungsbeispiele**

**Beispiel 1: Herstellung von O-Benzyl-N-hydroxypropylchitosan (Zweistufen-Verfahren)**

A) N-Hydroxypropylierung von Chitosan

50 g (0,31 mol) niedermolekulares Chitosan werden in einem Gemisch von 100 ml Ethanol oder Isopropanol und 100 ml Wasser dispergiert und im Autoklaven bei 100 Grad Celsius mit 104,5 g (1,8 mol) Propylenoxid 6 Stunden lang umgesetzt.

Nach beendeter Reaktion wird die Reaktionsmischung unter Nachspülen mit etwa 1 l Ethanol/Wasser (1 : 1) beziehungsweise Isopropanol/Wasser (1 : 1) aus dem Druckreaktor in einen Kolben überführt und im Vakuum auf ein Volumen von etwa 150 bis 200 ml eingeengt. Anschließend fällt man das N-Hydroxypropylchitosan in der 8 bis 10-fachen Menge Aceton aus.

Um eingeschlossene Propylenglykole zu entfernen, wird das gefällte Derivat sodann mittels eines Hochgeschwindigkeitsrührers fein dispergiert. Der feinflockige Niederschlag wird anschließend über eine G3-Glassinternutsche abgetrennt und so lange mit Aceton gewaschen bis das Filtrat farblos ist.

Nach dem Trocknen im Vakuumtrockenschrank bei 50 Grad Celsius erhält man 68 g wasserlösliches N-Hydroxypropylchitosan.

Kenndaten:

Grenzviskositätszahl (Eta): 80 ml/g
Substitutionsgrad Hydroxypropyl: 1,4 bis 1,6
Pendelhärte: 204 sec
Wasserdampfaufnahme: 8 Prozent

B) O-Benzylierung von N-Hydroxypropylchitosan

50 g (0,21 mol) N-Hydroxypropylchitosan aus Beispiel 1 A, 20,2 ml (0,32 mol) 43-prozentige Natronlauge sowie 235 ml Toluol werden im Autoklaven mit 53,16 g (0,48 mol) Benzylchlorid versetzt und 24 Stunden lang bei 90 Grad Celsius zur Reaktion gebracht.

Nach beendeter Reaktion verdünnt man mit Wasser, stellt den pH-Wert auf 7 ein und engt im Vakuum zur Trockene ein.

Das Umsetzungsprodukt wird in Aceton gelöst. Anschließend wird vom abgesetzten Salz abdekantiert und die erhaltene acetonische Lösung durch Zentrifugieren und Dekantieren geklärt. Sodann engt man die Lösung im Vakuum auf ein Volumen von etwa 100 ml ein und fällt das Chitosanderivat durch Zugabe von Wasser aus. Das Chitosanderivat wird bei 50 Grad Celsius im Vakuum getrocknet. Das so erhaltene Produkt wird in Petrolether zur Beseitigung des restlichen Benzylalkohols extrahiert.

Nach dem Absaugen über eine Glassinternutsche und dem erneuten Trocknen bei 50 Grad Celsius im Vakuum werden 67 g in organischen Lösungsmitteln lösliches O-Benzyl-N-hydroxypropylchitosan erhalten.

Kenndaten:

Grenzviskositätszahl (Eta): 27 ml/g
Substitutionsgrad Benzyl: 2,2
Substitutionsgrad Hydroxypropyl: 1,7
Pendelhärte: 173 sec
Wasserdampfaufnahme: 1,6 Prozent

**Beispiel 2: Herstellung von O-Benzyl-N-hydroxyethylchitosan ("Eintopf"-Verfahren)**

50 g (0,31 mol) niedermolekulares Chitosan werden in einem Gemisch bestehend aus 180 ml Ethylenglykoldimethylether und 20 ml destiliertem Wasser dispergiert und im Autoklaven mit insgesamt 79,3 g = 90 ml (1,8 mol) Ethylenoxid bei 40 Grad Celsius zur Reaktion gebracht. Die Zugabe des Ethylenoxids erfolgt hierbei portionsweise (jeweils 30 ml nach erfolgtem Druckabfall) über einen Zeitraum von etwa 2 Stunden.

Anschließend wird die Reaktionsmischung bei 40 Grad Celsius 6 Stunden lang gerührt und sodann das überschüssige Epoxid mittels Inertgas aus der Lösung entfernt. Die Reaktionsmischung wird mit 118,0 g (1,27 mol) 43-prozentige Natronlauge, 235,4 g = 214 ml (1,86 mol) Benzylchlorid sowie 200 ml Ethylenglykoldimethylether versetzt und bei 90 Grad Celsius 12 Stunden lang gerührt.

Die Aufarbeitung des organolöslichen Chitosanderivates erfolgt in der in Beispiel 1 beschriebenen Weise. Die Ausbeute an O-Benzyl-N-hydroxyethylchitosan beträgt 84 g.

Kenndaten:

Grenzviskositätszahl (Eta): 22 ml/g
Substitutionsgrad Benzyl: 3,5
Pendelhärte: 180 sec
Wasserdampfaufnahme: 1,2 Prozent

**Beispiel 3: Herstellung von hochmolekularem O-Benzyl-N-hydroxypropylchitosan (Zweistufen-Verfahren)**

A) N-Hydroxypropylierung von Chitosan

50 g (0,31 mol) hochmolekulares Chitosan werden in einem Gemisch von 100 ml Ethanol beziehungsweise Isopropanol und 100 ml Wasser dispergiert und im Autoklaven bei 100 Grad Celsius mit 104,5 g (1,8 mol) Propylenoxid 6 Stunden lang umgesetzt.

Nach beendeter Reaktion wird die Reaktionsmischung unter Nachspülen mit etwa 1 l Ethanol/Wasser (1 : 1) beziehungsweise Isopropanol/Wasser (1 : 1) aus dem Druckreaktor in einen Kolben überführt und im Vakuum auf ein Volumen von etwa 150 bis 200 ml eingeengt. Anschließend fällt man das N-Hydroxypropylchitosan in der 8 bis 10-fachen Menge Aceton aus.

Um eingeschlossene Propylenglykole zu entfernen, wird das gefällte Derivat sodann mittels eines Hochgeschwindigkeitsrührers fein dispergiert. Der feinflockige Niederschlag wird anschließend abfiltriert und so lange mit Aceton gewaschen bis das Filtrat farblos ist.

Nach dem Trocknen im Vackuum bei 50 Grad Celsius erhält man 71 g wasserlösliches N-Hydroxypropylchitosan.

Kenndaten:

Grenzviskositätszahl: 990 ml/g
Substitutionsgrad Hydroxypropyl: 2,0
Pendelhärte: 180 sec
Wasserdampfaufnahme: 5,4 Prozent

B) O-Benzylierung von N-Hydroxypropylchitosan

50 g (0,21 mol) N-Hydroxypropylchitosan aus Beispiel 3 A, 20,2 ml (0,32 mol) 43-prozentige Natronlauge sowie 235 ml Toluol werden im Autoklaven mit 53,16 g (0,16 mol) Benzylchlorid versetzt und 24 Stunden lang bei 90 Grad Celsius zur Reaktion gebracht.

Nach beendeter Reaktion verdünnt man mit Wasser, stellt den pH-Wert auf 7 ein und engt zur Trockne ein.

Das Umsetzungsprodukt wird in Aceton gelöst. Anschließend wird vom abgesetzten Salz abdekantiert und die erhaltene acetonische Lösung durch Zentrifugieren und Dekantieren geklärt. Sodann engt man die Lösung im Vakuum auf ein Volumen von etwa 100 ml ein und fällt das Chitosanderivat durch Zugabe von Wasser aus. Das Chitosanderivat wird bei 50 Grad Celsius im Vakuum getrocknet. Das so erhaltene Produkt wird zur Beseitigung des restlichen Benzylalkohols mit Petrolether extrahiert.

Nach dem Absaugen über eine Glassinternutsche und dem erneuten Trocknen bei 50 Grad Celsius im Vakuum werden 73 g O-Benzyl-N-hydroxypropylchitosan erhalten.

Kenndaten:

Grenzviskositätszahl: 600 bis 650 ml/g
Substitutionsgrad Benzyl: 1,8 bis 2,0
Substitutionsgrad Hydroxypropyl: 1,0 bis 1,2
Pendelhärte: 162 bis 170 sec
Wasserdampfaufnahme: 1,2 bis 1,4 Prozent

Als niedermolekulares Chitosan wurde ein gemahlenes Chitosan mit einer Grenzviskositätszahl (Eta) von 160 ml/g und einem Deacetylierungsgrad von 90 Prozent eingesetzt. Das verwendete hochmolekulare Chitosan hat eine Grenzviskositätszahl (Eta) von 1600 ml/g und einen Deacetylierungsgrad von 76 Prozent.

Der Substitutionsgrad für den Hydroxypropylrest beziehungsweise den Benzylrest wurde mit Hilfe der $^1$H-NMR-Spektroskopie ermittelt.

Die Messung der Grenzviskositätszahl (Eta) erfolgte in einer wäßrigen Lösung von 0,2 mol/l Essigsäure und 0,1 mol/l Natriumacetat (Chitosan) beziehungsweise in einer wäßrigen Lösung von 0,2 mol/l Essigsäure und 0,1 mol/l Natriumchlorid (N-Hydroxyalkylchitosan) beziehungsweise in Tetrahydrofuran (Benzylhydroxyalkylchitosan) bei 25 Grad Celsius unter Verwendung eines DIN-Ubbelohde-Kapillarviskosimeters.

Die Pendelhärte wurde nach König (W.König, "Härtemessungen mit dem Pendelhärteprüfer", Farbe und Lack 65, Seite 435 bis 443 (1959); DIN 53 157) bestimmt.

Die Wasserdampfaufnahme wurde bei 70 Prozent relativer Luftfeuchte gegenüber 30 Prozent relativer Luftfeuchte ermittelt.

## Beispiele für Nagellacke

### Beispiel 4 Farbloser Nagellack

| | |
|---|---|
| 15,0 g | O-Benzyl-N-hydroxyethylchitosan gemäß Beispiel 2 (Substitutionsgrad Benzyl = 3,5; Grenzviskositätszahl (Eta) = 22 ml/g) |
| 33,0 g | Methylenchlorid |
| 28,0 g | Ethylenglykolmonoethylether |
| 17,0 g | Ethanol |
| 5,0 g | Dibutylphthalat |
| 2,0 g | Diethylenglykolmonomethylether |
| 100,0 g | |

### Beispiel 5 Farbiger Nagellack

| | |
|---|---|
| 12,0 g | O-Benzyl-N-hydroxyethylchitosan gemäß Beispiel 2 (Substitutionsgrad Benzyl = 3,5; Grenzviskositätszahl (Eta) = 22 ml/g) |
| 28,0 g | Methylenchlorid |
| 20,0 g | Ethylenglykolmonomethylether |
| 14,5 g | Ethanol |
| 9,0 g | Ethylacetat |
| 6,0 g | Butylacetat |
| 4,0 g | Dibutylphthalat |
| 2,0 g | Diethylenglykolmonomethylether |
| 2,0 g | Trikresylphosphat |
| 2,5 g | Pigmente |
| 100,0 g | |

**Beispiel 6 Farbloser Nagellack**

| | |
|---|---|
| 6,0 g | O-Benzyl-N-hydroxypropylchitosan gemäß Beispiel 1 (Substitutionsgrad Benzyl = 2,2; Grenzviskositätszahl (Eta) = 27 ml/g) |
| 40,0 g | Butylacetat |
| 30,0 g | Ethylacetat |
| 18,0 g | Nitrocellulose (alkoholfeucht 65:35) |
| 4,0 g | Dibutylphthalat |
| 2,0 g | Campher |
| 100,0 g | |

**Beispiel 7 Farbiger Nagellack**

| | |
|---|---|
| 8,0 g | O-Benzyl-N-hydroxypropylchitosan gemäß Beispiel 1 (Substitutionsgrad Benzyl 2,2; Grenzviskositätszahl (Eta) = 27 ml/g) |
| 35,0 g | Ethylenglykolmonomethylether |
| 21,5 g | Methylenchlorid |
| 15,0 g | Aceton |
| 12,0 g | Nitrocellulose (alkoholfeucht 65:35) |
| 6,0 g | Dibutylphthalat |
| 1,5 g | Pigmente |
| 1,0 g | Sedimentationsverzögerer |
| 100,0 g | |

**Beispiel 8 Farbloser Nagellack**

| | |
|---|---|
| 12,0 g | O-Benzyl-N-hydroxypropylchitosan gemäß Beispiel 2 (Substitutionsgrad Benzyl = 1,8 bis 2,0; Grenzviskositätszahl (Eta) = 600 bis 650 ml/g) |
| 41,0 g | Methylenchlorid |
| 15,0 g | Ethylenglykolmonomethylether |
| 15,0 g | Butylacetat |
| 12,0 g | Ethylacetat |
| 5,0 g | Diethylenglykolmonomethylether |
| 100,0 g | |

Als Nitrocellulose wird eine esterlösliche Nitrocellulose verwendet, deren Viskosität in Aceton (5 Gewichtsprozent Wassergehalt), bei einem Gehalt von 22 Gewichtsprozent Nitrocellulose, 400 ± 25 mPas beträgt. Die Bestimmung der Viskosität erfolgt hierbei nach DIN 53 179, bei einer Temperatur von 20 ± 0,05 Grad Celsius, mit einem Kugelfallviskosimeter nach Höppler (thermostatisierbares Fallrohr mit einem Durchmesser von 15,94 mm; Neigung des Fallrohres gegen die Senkrechte: 10 ± 0,1 Grad; Länge der Meßstrecke: 100 mm) unter Verwendung von Kugel Nr. 4 (Werkstoff: Nickel-Eisen; Kugeldurchmesser: 15,2 ± 0,1 mm; K = 0,7 mPas•cm$^3$). Beispiele für eine derartige Nitrocellulose sind "Walsroder Collodiumwolle E 560" der Wolff Walsrode AG, Walsrode und "Nitrocellulose RS 1/2 Sekunde" der Hercules Inc., Wilmington/USA.

Alle in der vorliegenden Anmeldung genannten Prozentzahlen stellen Gewichtsprozente dar.

## Patentansprüche

### Patentansprüche für folgende Vertragsstaaten : DE, FR, GB, IT

**1.** Nagellack auf der Basis eines Filmbildners, einer Harzkomponente und eines Lösungsmittelsystems, dadurch gekennzeichnet, daß er als Filmbildner und/oder Harzkomponente ein O-Benzyl-N-hydroxyalkylchitosan der allgemeinen Formel (I)

$$HO\ [\ C_6H_{11\text{-}m\text{-}q}NO_4\ (R^1)_m(R^2)_n(R^3)_q\ ]_p\ H \qquad (I),$$

wobei m jeden beliebigen Zahlenwert von 0 bis 0,6,
n jeden beliebigen Zahlenwert von 0,1 bis 10
und q jeden beliebigen Zahlenwert von 0,1 bis 4
annehmen kann,
p für eine ganze Zahl von 50 bis 5.000 steht,
$R^1$ den Rest

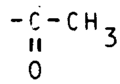

bedeutet,
$R^2$ den Rest

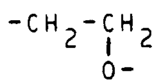 , 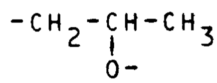 oder

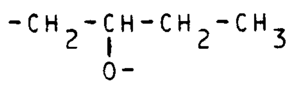

darstellt
und $R^3$ den Rest

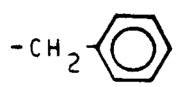

bedeutet, enthält.

**2.** Nagellack nach Anspruch 1, dadurch gekennzeichnet, daß das O-Benzyl-N-hydroxyalkylchitosan der Formel (I) ein durchschnittliches Molekulargewicht von 5.000 bis 1.800.000, bevorzugt von 10.000 bis 500.000, aufweist.

**3.** Nagellack nach Anspruch 1 oder 2, dadurch gekennzeichnet, daß die Grenzviskositätszahl des O-Benzyl-N-hydroxyalkylchitosans der Formel (I) in Tetrahydrofuran bei 25 Grad Celsius in einem Bereich von 5 bis 200 ml/g, vorzugsweise von 10 bis 120 ml/g, liegt.

**4.** Nagellack nach einem der Ansprüche 1 bis 3, dadurch gekennzeichnet, daß der Benzyl-Substitutionsgrad des O-Benzyl-N-hydroxyalkylchitosans der Formel (I) 0,8 bis 3,8 beträgt.

**5.** Nagellack nach einem der Ansprüche 1 bis 4, dadurch gekennzeichnet, daß das O-Benzyl-N-hydroxyalkylchitosan der Formel (I) in einer Konzentration von 3 bis 30 Gewichtsprozent enthalten ist.

**6.** Nagellack nach einem der Ansprüche 1 bis 5, dadurch gekennzeichnet, daß das Lösungsmittelsystem aus einer Mischung niedrig-, mittel- und hochsiedender organischer Lösungsmittel besteht.

7. Nagellack nach einem der Ansprüche 1 bis 6, dadurch gekennzeichnet, daß das Lösungsmittelsystem 60 bis 80 Gewichtsprozent des Nagellacks ausmacht.

8. Nagellack nach einem der Ansprüche 1 bis 7, dadurch gekennzeichnet, daß er zusätzlich mindestens ein organisches oder anorganisches Pigment in einer Menge von 0,1 bis 6 Gewichtsprozent enthält.

9. Nagellack nach einem der Ansprüche 1 bis 8, dadurch gekennzeichnet, daß er zusätzlich ein die Sedimentation verzögerndes Mittel enthält.

10. Makromolekulare, vom Chitosan abgeleitete 0-Benzyl-N-hydroxyalkylverbindung der allgemeinen Formel (I)

$HO\ [\ C_6H_{11-m-q}\ NO_4\ (R^1)_m(R^2)_n(R^3)_q\ ]_p\ H$ (I),

wobei m jeden beliebigen Zahlenwert von 0 bis 0,6,
n jeden beliebigen Zahlenwert von 0,1 bis 10
und q jeden beliebigen Zahlenwert von 0,1 bis 4
annehmen kann,
p für eine ganze Zahl von 50 bis 5.000 steht,
$R^1$ den Rest

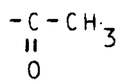

bedeutet,
$R^2$ den Rest

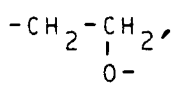, 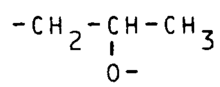 oder

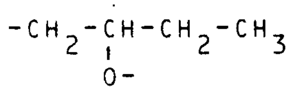

darstellt
und $R^3$ den Rest

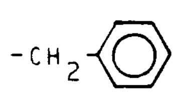

bedeutet.

**Patentansprüche für folgenden Vertragsstaat : ES**

1. Nagellack auf der Basis eines Filmbildners, einer Harzkomponente und eines Lösungsmittelsystems, dadurch gekennzeichnet, daß er als Filmbildner und/oder Harzkomponente ein O-Benzyl-N-hydroxyalkylchitosan der allgemeinen Formel (I)

$HO\ [\ C_6H_{11-m-q}\ NO_4\ (R^1)_m(R^2)_n(R^3)_q\ ]_p\ H$ (I),

wobei m jeden beliebigen Zahlenwert von 0 bis 0,6, n jeden beliebigen Zahlenwert von 0,1 bis 10 und q jeden beliebigen Zahlenwert von 0,1 bis 4 annehmen kann,
p für eine ganze Zahl von 50 bis 5.000 steht,
$R^1$ den Rest

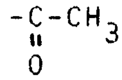

bedeutet,
$R^2$ den Rest

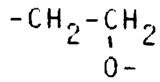 ,

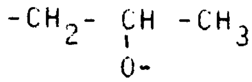

oder 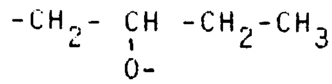

darstellt
und $R^3$ den Rest

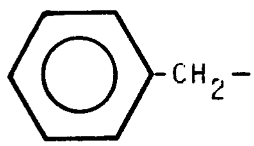

bedeutet, enthält.

**2.** Nagellack nach Anspruch 1, dadurch gekennzeichnet, daß das O-Benzyl-N-hydroxyalkylchitosan der Formel (I) ein durchschnittliches Molekulargewicht von 5.000 bis 1.800.000, bevorzugt von 10.000 bis 500.000, aufweist.

**3.** Nagellack nach Anspruch 1 oder 2, dadurch gekennzeichnet, daß die Grenzviskositätszahl des O-Benzyl-N-hydroxyalkylchitosans der Formel (I) in Tetrahydrofuran bei 25 Grad Celsius in einem Bereich von 5 bis 200 ml/g, vorzugsweise von 10 bis 120 ml/g, liegt.

**4.** Nagellack nach einem der Ansprüche 1 bis 3, dadurch gekennzeichnet, daß der Benzyl-Substitutionsgrad des O-Benzyl-N-hydroxyalkylchitosan, der Formel (I) 0,8 bis 3,8 beträgt.

**5.** Nagellack nach einem der Ansprüche 1 bis 4, dadurch gekennzeichnet, daß das O-Benzyl-N-hydroxyalkylchitosan der Formel (I) in einer Konzentration von 3 bis 30 Gewichtsprozent enthalten ist.

**6.** Nagellack nach einem der Ansprüche 1 bis 5, dadurch gekennzeichnet, daß das Lösungsmittelsystem aus einer Mischung niedrig-, mittel- und hochsiedender organischer Lösungsmittel besteht.

**7.** Nagellack nach einem der Ansprüche 1 bis 6, dadurch gekennzeichnet, daß das Lösungsmittelsystem 60 bis 80 Gewichtsprozent des Nagellacks ausmacht.

**8.** Nagellack nach einem der Ansprüche 1 bis 7, dadurch gekennzeichnet, daß er zusätzlich mindestens ein organisches oder anorganisches Pigment in einer Menge von 0,1 bis 6 Gewichtsprozent enthält.

**9.** Nagellack nach einem der Ansprüche 1 bis 8, dadurch gekennzeichnet, daß er zusätzlich ein die Sedimentation verzögerndes Mittel enthält.

**10.** Verfahren zur Herstellung von makromolekularen, vom Chitosan abgeleiteten O-Benzyl-N-hydroxyalkylverbindungen der allgemeinen Formel (I)

$HO\,[\,C_6H_{11\text{-}m\text{-}q}\,NO_4\,(R^1)_m(R^2)_n(R^3)_q\,]_p\,H$ (I),

wobei m jeden beliebigen Zahlenwert von 0 bis 0,6,
n jeden beliebigen Zahlenwert von 0,1 bis 10
und q jeden beliebigen Zahlenwert von 0,1 bis 4
annehmen kann,
p für eine ganze Zahl von 50 bis 5.000 steht,
$R^1$ den Rest

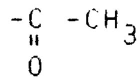

bedeutet.
$R^2$ den Rest

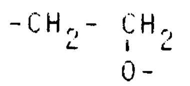
,

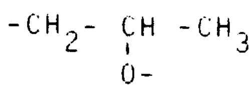

oder 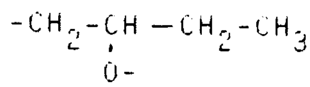

darstellt
und $R^3$ den Rest

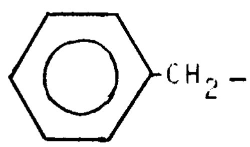

bedeutet, dadurch gekennzeichnet, daß man Chitosan in einer ersten Reaktionsstufe in Gegenwart eines geeigneten Dispergiermittels bei einer Temperatur von 20 bis 120 Grad Celsius 6 bis 60 Stunden lang mit einem $C_2$ bis $C_4$ - Epoxid umsetzt und anschließend das erhaltene N-Hydroxyalkylchitosan in einer zweiten Reaktionsstufe mit Benzylchlorid im alkalischen Medium bei 40 bis 120 Grad Celsius 6 bis 60 Stunden lang zur Umsetzung bringt.

**11.** Verfahren zur Herstellung von makromolekularen, vom Chitosan abgeleiteten O-Benzyl-N-hydroxyalkylverbindungen der allgemeinen Formel (I)

$HO\,[\,C_6H_{11\text{-}m\text{-}q}\,NO_4\,(R^1)_m(R^2)_n(R^3)_q\,]_p\,H$ (I),

wobei m jeden beliebigen Zahlenwert von 0 bis 0,6,
n jeden beliebigen Zahlenwert von 0,1 bis 10
und q jeden beliebigen Zahlenwert von 0,1 bis 4
annehmen kann,
p für eine ganze Zahl von 50 bis 5.000 steht,
$R^1$ den Rest

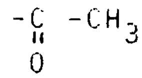

bedeutet,
$R^2$ den Rest

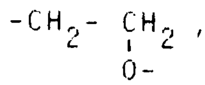

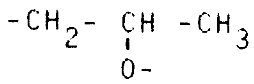

oder 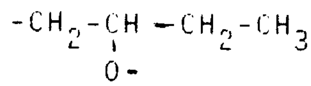

darstellt
und $R^3$ den Rest

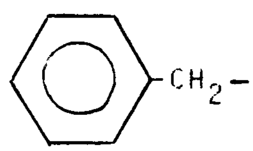

bedeutet, dadurch gekennzeichnet, daß man Chitosan gemeinsam mit einem $C_2$ bis $C_4$ - Epoxid sowie Benzylchlorid im Autoklaven 6 bis 60 Stunden lang bei 20 bis 120 Grad Celsius rührt, sodann die Reaktionsmischung auf einen alkalischen pH-Wert einstellt und bei 40 bis 120 Grad Celsius weitere 6 bis 60 Stunden lang rührt.

**12.** Verfahren gemäß Anspruch 10 oder 11, dadurch gekennzeichnet, daß das molare Verhältnis von Chitosan zu Alkylierungsmittel zwischen 1 : 3 und 1 : 5 liegt.

## Claims

### Claims for the following Contracting States : DE, FR, GB, IT

**1.** Nail varnish based on a film forming component, a resin constituent and a solvent system, characterised in that as a film forming component and/or a resin constituent, it contains an O-benzyl-N-hydroxyalkyl chitosan of the general formula (I)

$$HO\,[\,C_6H_{11-m-q}NO_4\,(R^1)_m(R^2)_n(R^3)_q\,]_p\,H \qquad (I),$$

wherein m can assume any value from 0 to 0.6,
n can assume any value from 0.1 to 10
and q can assume any value from 0.1 to 4,
p stands for an integer from 50 to 5.000,
$R^1$ is the residue

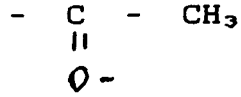

$R^2$ is the residue

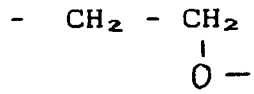

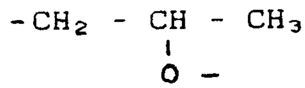

or 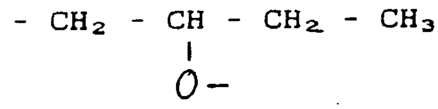

and $R^3$ is the residue

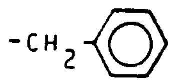

2. Nail varnish according to Claim 1, characterised in that the O-benzyl-N-hydroxyalkyl chitosan of the general formula (I) has an average molecular weight of 5,000 to 1,800.000, preferably from 10,000 to 500,000.

3. Nail varnish according to Claim 1 or 2, characterised in that the limiting viscosity number of the O-benzyl-N-hydroxyalkyl chitosan of the formula (I) in tetrahydrofuran at 25° Celsius is in the region of 5 to 200 ml/g, preferably from 10 to 120 ml/g.

4. Nail varnish according to any one of the Claims 1 to 3, characterised in that the degree of benzyl-substitution of the O-benzyl-N-hydroxyalkyl chitosan of the formula (I) amounts to 0.8 to 3.8.

5. Nail varnish according to any one of the Claims 1 to 4, characterised in that the O-benzyl-N-hydroxyalkyl chitosan of the formula (I) is present in a concentration of 3 to 30 weight %.

6. Nail varnish according to any one of the Claims 1 to 5, characterised in that the solvent system consists of a mixture of low-, medium-, and high boiling point organic solvents.

7. Nail varnish according to any one of Claims 1 to 6, characterised in that the solvent system makes up 60 to 80 weight % of the nail varnish.

8. Nail varnish according to any one of the Claims 1 to 7, characterised in that it also contains at least one organic or inorganic pigment in a quantity of 0.1 to 6 weight %.

9. Nail varnish according to any one of the Claims 1 to 8, characterised in that it also contains a sedimentation retarding agent.

10. Macromolecular O-benzyl-N-hydroxyalkyl compound derived from chitosan of the general formula (I)

$HO [ C_6 H_{11-m-q} NO_4 (R^1)_m(R^2)_n(R^3)_q ]_p H$ (I),

wherein m can assume any value from 0 to 0.6,
n can assume any value from 0.1 to 10
and q can assume any value from 0.1 to 4,
p stands for an integer between 50 and 5.000,
$R^1$ means the residue

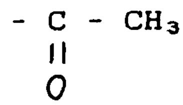

$R^2$ represents the residue

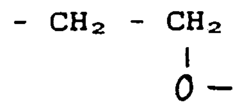

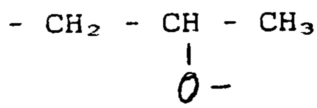

or 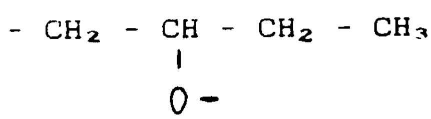

and $R^3$ means the residue

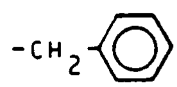

**Claims for the following Contracting State : ES**

1. Nail varnish based on a film forming component, a resin constituent and a solvent system, characterised in that as a film forming component and/or a resin constituent, it contains an O-benzyl-N-hydroxyalkyl chitosan of the general formula (1)

$HO\,[\,C_6\,H_{11\text{-}m\text{-}q}\,NO_4\,(R^1)_m(R^2)_n(R^3)_q\,]_p\,H$ (1),

wherein m can assume any value from 0 to 0.6,
n can assume any value from 0.1 to 10
and q can assume any value from 0.1 to 4,
p stands for an integer from 50 to 5.000,
$R^1$ is the residue

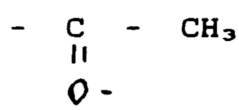

$R^2$ is the residue

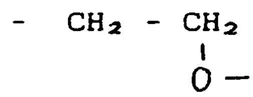

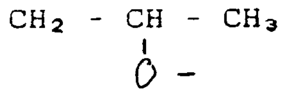

or 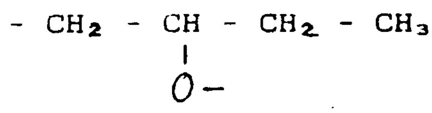

and $R^3$ is the residue

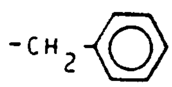

2. Nail varnish according to Claim 1, characterised in that the O-benzyl-N-hydroxyalkyl chitosan of the general formula (1) has an average molecular weight of 5,000 to 1,800,000, preferably from 10,000 to 500,000.

3. Nail varnish according to Claim 1 or 2, characterised in that the limiting viscosity number of the O-benzyl-N-hydroxyalkyl chitosan of the formula (1) in tetrahydrofuran at 25° Celsius is in the region of 5 to 200 ml/g, preferably from 10 to 120 ml/g.

4. Nail varnish according to any one of the Claims 1 to 3, characterised in that the degree of benzyl-substitution of the O-benzyl-N-hydroxyalkyl chitosan of the formula (1) amounts to 0.8 to 3.8.

5. Nail varnish according to any one of the Claims 1 to 4, characterised in that the O-benzyl-N-hydroxyalkyl chitosan of the formula (1) is present in a concentration of 3 to 30 weight %.

6. Nail varnish according to any one of the Claims 1 to 5, characterised in that the solvent system consists of a mixture of low-, medium-, and high boiling point organic solvents.

7. Nail varnish according to any one of Claims 1 to 6, characterised in that the solvent system makes up 60 to 80 weight % of the nail varnish.

8. Nail varnish according to any one of the Claims 1 to 7, characterised in that it also contains at least one organic or inorganic pigment in a quantity of 0.1 to 6 weight %.

9. Nail varnish according to any one of the Claims 1 to 8, characterised in that it also contains an sedimentation retarding agent.

10. Process for the production of macromolecular O-benzyl-N-hydroxyalkyl compounds derived from chitosan of the general formula (1)

$$HO\ [\ C_6 H_{11\text{-}m\text{-}q}\ NO_4\ (R^1)_m (R^2)_n (R^3)_q\ ]_p\ H \qquad (1),$$

wherein m can assume any value from 0 to 0.6,
n can assume any value from 0.1 to 10
and q can assume any value from 0.1 to 4,
p stands for an integer between 50 and 5.000,
$R^1$ means the residue

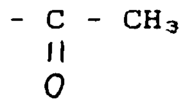

$R^2$ represents the residue

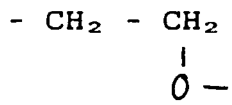

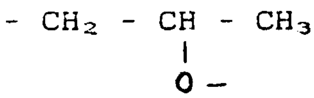

or 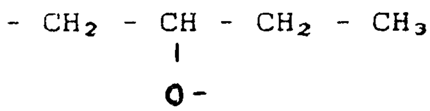

and $R^3$ means the residue

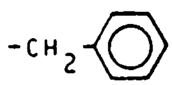 ,

characterized in that in a first reaction step chitosan is reacted in the presence of a suitable dispersing agent with a $C_2$ - $C_4$-epoxide for 6 to 60 hours at a temperature of 20 to 120 °C and the N-hydroxyalkylchitosan obtained is reacted in a second reaction step with benzyl chloride in alkaline medium for 6 to 60 hours at a temperature of 40 to 120 °C.

**11.** Process for the production of macromolecular O-benzyl-N-hydroxyalkyl compounds derived from chitosan of the general formula (1)

$$HO\,[\,C_6H_{11-m-q}NO_4\,(R^1)_m(R^2)_n(R^3)_q\,]_p\,H \qquad (1),$$

wherein m can assume any value from 0 to 0.6,
n can assume any value from 0.1 to 10
and q can assume any value from 0.1 to 4,
p stands for an integer between 50 and 5.000,
$R^1$ means the residue

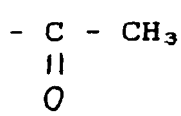

$R^2$ represents the residue

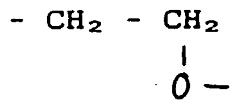

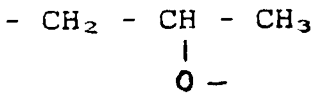

or 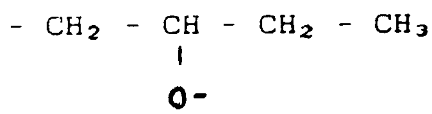

and $R^3$ means the residue

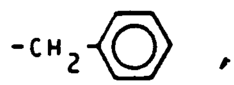 ,

characterized in that chitosan is reacted in an autoclave with a $C_2$- to $C_4$-epoxide and benzyl chloride for 6 to 60 hours at a temperature of 20 to 120 °C, then the reaction mixture is adjusted to an alcaline pH-value and stirred for another 6 to 60 hours at 40 to 120 °C.

**12.** Process according to Claim 10 or 11, characterized in that the molar ratio of chitosan to alkylizing agent is between 1:3 and 1:5.

**Revendications**

**Revendications pour les Etats contractants suivants : DE, FR, GB, IT**

**1.** Vernis à ongles à base d'un filmogène, d'un constituant en résine et d'un système de solvants, caractérisé en ce qu'il renferme, somme filmogène et/ou constituant en résine, un O-benzyl-N-hydroxyalkylchitosane répondant à la formule générale (I)

$HO [ C_6 H_{11-m-q} NO_4 (R^1)_m(R^2)_n(R^3)_q ]_p H$ (I),

dans laquelle m peut prendre n'importe quelle valeur numérique de 0 à 0,6, n n'importe quelle valeur numérique de 0,1 à 10 et q n'importe quelle valeur numérique de 0,1 à 4,
p représente un nombre entier de 50 à 5.000,
$R^1$ représente le reste

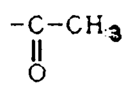

$R^2$ représente le reste

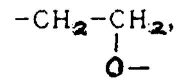

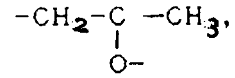

ou 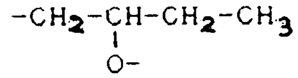

et $R^3$ le reste

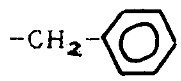

**2.** Vernis à ongles selon la revendication 1, caractérisé en ce que le O-benzyl-N-hydroxyalkylchitosane répondant à la formule générale (I) a un poids moléculaire moyen de 5.000 à 1,800,000, de préférence de 10.000 à 500.000.

**3.** Vernis à ongles selon la revendication 1 ou 2, caractérisé en ce que l'indice de viscosité limite du O-benzyl-N-hydroxyalkylchitosane répondant à la formule générale (I) dans le tétrahydrofuranne à 25 degrés Celsius est compris dans un intervalle de 5 à 200 ml/g, de préférence de 10 à 120 ml/g.

**4.** Vernis à ongles selon l'une des revendications 1 à 3, caractérisé en ce que le degré de substitution benzylique du O-benzyl-N-hydroxyalkylchitosane répondant à la formule générale (I) est de 0,8 à 3,8.

**5.** Vernis à ongles selon l'une des revendications 1 à 4, caractérisé en ce que le O-benzyl-N-hydroxyalkylchitosane répondant à la formule (I) est contenu à une concentration de 3 à 30 % en poids.

**6.** Vernis à ongles selon l'une des revendications 1 à 5, caractérisé en ce que le système de solvants consiste en un mélange de solvants organiques à points d'ébullition bas, moyens et élevés.

**7.** Vernis à ongles selon l'une des revendications 1 à 6, caractérisé en ce que le système de solvants représente 60 à 80 % en poids du vernis à ongles.

**8.** Vernis à ongles selon l'une des revendications 1 à 7, caractérisé en ce qu'il renferme en plus au moins un pigment organique ou inorganique dans une porportion de 0,1 à 6 % en poids.

**9.** Vernis à ongles selon l'une des revendications 1 à 8, caractérisé en ce qu'il renferme, en plus, un produit ralentissant la sédimentation.

**10.** Composé O-benzyl-N-hydroxyalkyl macromoléculaire, dérivant du chitosane, répondant à la formule générale (I)

$HO\,[\,C_6H_{11\text{-}m\text{-}q}\,NO_4\,(R^1)_m(R^2)_n(R^3)_q\,]_p\,H$ (I),

dans laquelle m peut prendre n'importe quelle valeur numérique de 0 à 0,6, n n'importe quelle valeur numérique de 0,1 à 10 et q n'importe quelle valeur numérique de 0,1 à 4,
p représente un nombre entier de 50 à 5.000,
$R^1$ représente le reste

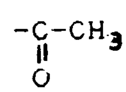

$R^2$ représente le reste

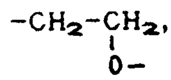

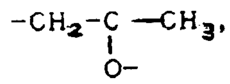

ou 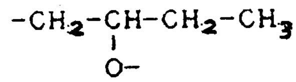

et $R^3$ le reste

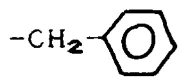 .

**Revendications pour l'Etats contractant suivant : ES**

**1.** Vernis à ongles à base d'un filmogène, d'un constituant en résine et d'un système de solvants, caractérisé en ce qu'il renferme, comme filmogène et/ou constituant en résine, un O-benzyl-N-hydroxyalkylchitosane répondant à la formule générale (I)

$HO\ [\ C_6H_{11\text{-}m\text{-}q}\ NO_4\ (R^1)_m(R^2)_n(R^3)_q\ ]_p\ H$ (I),

dans laquelle m peut prendre n'importe quelle valeur numérique de 0 à 0,6, n n'importe quelle valeur numérique de 0,1 à 10 et q n'importe quelle valeur numérique de 0,1 à 4,
p représente un nombre entier de 50 à 5.000,
$R^1$ représente le reste

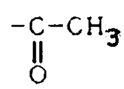

$R^2$ représente le reste

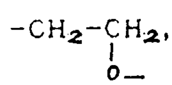

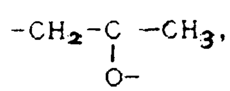

ou 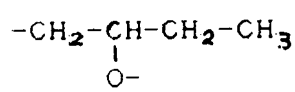

et $R^3$ le reste

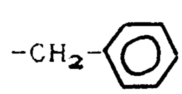

2. Vernis à ongles selon le revendication 1, caractérisé en ce que le O-benzyl-N-hydroxyalkylchitosane répondant à le formule générale (I) a un poids moléculaire moyen de 5.000 à 1.800.000, de préférence de 10.000 à 500.000.

3. Vernis à ongles selon la revendication 1 ou 2, caractérisé en ce que l'indice de viscosité limite du O-benzyl-N-hydroxyalkylchitosane répondant à le formule générale (I) dans le tétrahydrofuranne à 25 degrés Celsius est compris dans un intervalle de 5 à 200 ml/g, de préférence de 10 à 120 ml/g.

4. Vernis à ongles selon l'une des revendications 1 à 3, caractérisé en ce que le degré de substitution benzylique du O-benzyl-N-hydroxyalkylchitosane répondant à la formule générale (I) est de 0,8 à 3,8.

5. Vernis à ongles selon l'une des revendications 1 à 4, caractérisé en ce que le 0-benzyl-N-hydroxyalkyl-chitosane répondant à la formule (I) est contenu à une concentration de 3 à 30 % en poids.

6. Vernis à ongles selon l'une des revendications 1 à 5, caractérisé en ce que le système de solvants consiste en un mélange de solvants organiques à points d'ébullition bas, moyens et élevés.

7. Vernis à ongles selon l'une des revendications 1 à 6, caractérisé en ce que le système de solvants représente 60 à 80 % en poids du vernis à ongles.

8. Vernis à ongles selon l'une des revendications 1 à 7, caractérisé en ce qu'il renferme en plus au moins un pigment organique ou inorganique dans une porportion de 0,1 à 6 % en poids.

9. Vernis à ongles selon l'une des revendications 1 à 8, caractérisé en ce qu'il renferme, en plus, un produit ralentissant la sédimentation.

10. Procédé de préparation des Composé O-benzyl-N-hydroxyalkylique macromoléculaire, dérivant du chitosane, répondant à la formule générale (I)

$$HO\ [\ C_6H_{11-m-q}NO_4\ (R^1)_m(R^2)_n(R^3)_q\ ]_p\ H \qquad (I),$$

dans laquelle m peut prendre n'importe quelle valeur numérique de 0 à 0,6, n n'importe quelle valeur numérique de 0,1 à 10 et q n'importe quelle valeur numérique de 0,1 à 4,
p représente un nombre entier de 50 à 5.000,
$R^1$ représente le reste

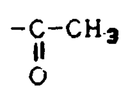

$R^2$ représente le reste

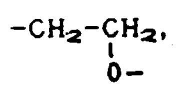

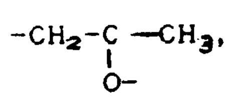

ou 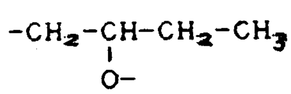

et $R^3$ le reste

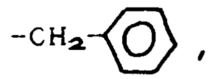

characérisé en ce qu'on fait réagir un chitosane au cours d'un premier stade de réaction, en présence d'un agent dispersant approprié, à une tepérature de 20 à 120 °C pour 6 à 60 heures sur un $C_2$ - $C_4$-epoxyde et l'on fait réagir le N-hydroxyalkyl-chitosane obtenu, au cours d'un second stade de réaction, sur le chlorure de benzyle, au milieu alcalin, à une température de 40 à 120 °C pour 6 à 60 heures.

**11.** Procédé de préparation des Composés O-benzyl-N-hydroxyalkylique macromoléculaire, dérivant du chitosane, répondant à la formule générale (I)

$HO\ [\ C_6H_{11\text{-}m\text{-}q}\ NO_4\ (R^1)_m(R^2)_n(R^3)_q\ ]_p\ H$ (I),

dans laquelle m peut prendre n'importe quelle voleur numérique de 0 à 0,6, n n'importe quelle valeur numérique de 0,1 à 10 et q n'importe quelle voleur numérique de 0,1 à 4,
p représente un nombre entier de 50 à 5.000,
$R^1$ représente le reste

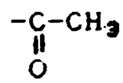

$R^2$ représente le reste

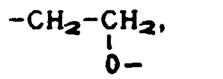

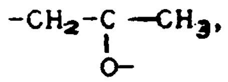

ou 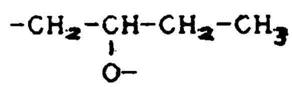

et $R^3$ le reste

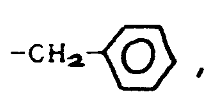

charactérisé en ce qu'on fait réagir un chitosan sur un $C_2$ - $C_4$ - epoxyde et de chlorure de benzyle dans un autoclave à une température de 20 à 120°C pour 6 à 60 heures et après alcaliniser la mélange l'on fait agiter à une température de 40 à 120 °C pour 6 à 60 heures.

**12.** Procédé selon la revendication 10 ou 11, charactérisé en ce que la proportion molaire de chitosane à l'agent d'alkylation est entre 1:3 et 1:5.